# EUROPEAN PATENT SPECIFICATION

(11) **EP 2 058 015 B1**
(45) Date of publication and mention of the grant of the patent: **08.01.2014**
(21) Application number: 08253618.6
(22) Date of filing: 05.11.2008
(51) Int. Cl.: A61L 27/50, A61L 31/14

(54) **Novel surgical fastener**
Neuartige chirurgische Befestigung
Nouvelle attache chirurgicale

(30) Priority: 05.11.2007 US 985358 P; 27.10.2008 US 258621
(43) Date of publication of application: 13.05.2009
(73) Proprietor: Covidien LP, Mansfield, MA 02048 (US)
(72) Inventor: Bettuchi, Michael, Middletown, CT 06457 (US); Heinrich, Russell, Madison, CT 06443 (US)
(74) Representative: Soames, Candida Jane

(56) References cited:
- EP-A2- 0 326 426
- WO-A1-03/088846
- WO-A2-03/088818
- US-A- 5 258 000

## Description

### TECHNICAL FIELD

The present disclosure relates generally to surgical devices for repairing body tissue, including fasteners capable of fastening objects to body tissue. In embodiments, the present disclosure provides a surgical fastener capable of changing its shape in vivo to secure tissue or a device; such as a surgical mesh, to tissue. Methods for using such fasteners are also provided.

### BACKGROUND

A number of surgical procedures require instruments that are capable or applying a surgical fastener to tissue in order to form tissue connections or to secure objects to tissue. For example, during hernia repair it is often desirable to fasten a surgical mesh to the underlying body tissue. In certain hernias, such as direct or indirect inguinal hernias, a part of the intestine protrudes through a defect or an opening in the supporting abdominal wall to form a hernial sac. The opening can be repaired using an open surgery procedure in which a relatively large incision is made in the patient and the hernia is closed off outside the abdominal wall by suturing. Alternatively, a mesh may be attached with sutures over the opening to provide reinforcement.

Less invasive surgical procedures are currently available for hernia repair. In laparoscopic procedures, surgery is performed in the abdomen through a small incision, while in endoscopic procedures surgery is performed through narrow endoscopic tubes inserted through small incisions in the body. Laparoscopic and endoscopic procedures generally require long and narrow instruments capable of reaching deep within the body and configured to form a seal with the incision or tube through which they are inserted.

Currently, endoscopic techniques for hernia repair utilize fasteners, such as surgical staples or clips, to secure a mesh to tissue in order to provide reinforcement to the repair and in order to provide structure for encouragement of tissue ingrowth. These staples or clips need to be compressed against the tissue and mesh in order to secure the two together thereby requiring a tool which is positioned on each side of the mesh and tissue in order to deform the staple or clip. Another type of fastener suited for use in affixing mesh to tissue, during procedures such as hernia repair, is a coil fastener having a helically coiled body portion terminating in a tissue penetrating tip, which helical fastener is screwed into the mesh and body tissue. An example of this type of fastener is disclosed in U.S. Pat. No. 5,258,000. Devices which introduce elongated wire fasteners and are capable of imparting shapes, for example annular shapes, to the wire as it is introduced into a patient's body are also known. Examples of such devices are disclosed in U.S. Patent Application Publication No. 2003/0236534.

Means for securing tissue and devices thereto, including surgical meshes utilized for hernia repair, and the use of such means in less invasive procedures, remain desirable.

EP 0 326 426 A2 discloses plastic molded articles, such as staples or clips for tissue suturing. The articles have shape memory properties.

WO 03/088818 A2 discloses shape memory polymers that are capable of changing their shape after an increase in temperature. Their shape memory capability enables bulky implants to be placed in the body through small incisions or to perform complex mechanical deformations automatically. In a preferred embodiment the implant is a suture.

### SUMMARY

The present disclosure provides fasteners for use in surgical procedures. The surgical fastener of the present invention includes an elongated shaft formed of a shape memory polymeric material having a pointed distal end, and a proximal end, wherein the surgical fastener possesses a temporary shape permitting insertion of the surgical fastener into tissue and a permanent shape capable of affixing the surgical fastener to tissue. In embodiments, a fastener of the present disclosure may also be utilized to affix an optional medical device to tissue.

The fastener of the present invention has a straight temporary shape and a permanent shape as described in claim 1.

Methods for securing medical devices, in embodiments surgical meshes, with fasteners of the present disclosure are also provided. In embodiments, suitable methods include providing a surgical fastener of the present disclosure in a temporary shape which facilitates its insertion into tissue, advancing the surgical fastener through a medical device and tissue, heating the fastener, and allowing the fastener to revert to a permanent shape thereby affixing the medical device to tissue.

### BRIEF DESCRIPTION OF THE DRAWINGS

FIG. 1 is a depiction of a surgical fastener of the present disclosure made of a shape memory polymer in its temporary shape;

FIG. 2 is a depiction of a surgical fastener of the present disclosure made of a shape memory polymer in its permanent shape;

FIG. 3 is a depiction of a surgical fastener of the present disclosure in its temporary shape after having been passed through a mesh and tissue; and

FIG. 4 is a depiction of a surgical fastener of the present disclosure in its permanent shape affixing a mesh to tissue.

### DETAILED DESCRIPTION

The present disclosure provides a surgical fastener formed of a shape memory polymeric material which is capable of adopting a shape in vivo suitable for adhering tissue or affixing a surgical device, including a hernia mesh, to tissue. Shape memory polymeric materials utilized to form a fastener of the present disclosure possess a permanent shape and a temporary shape. In embodiments, the temporary shape is of a configuration which enhances the ability for the surgeon to introduce a surgical fastener into a patient's body. The permanent shape, which is assumed in vivo upon application of heat, such as body heat, is of a configuration which enhances the retention of the surgical fastener in tissue and/or adhering a device such as a mesh to tissue. In embodiments, the surgical fastener possesses a straight temporary shape which facilitates its insertion into tissue, and a permanent shape which may, in embodiments, be curved or helical to secure tissue or affix a device to tissue.

In embodiments the surgical fastener may possess a curved temporary shape and a curved permanent shape possessing greater curvature than the temporary shape. In other embodiments the surgical fastener may possess a curved temporary shape and a helical permanent shape. In some embodiments surgical fasteners of the present disclosure may possess barbs oriented on the surface thereof which permit insertion into tissue but not removal therefrom. Surgical fasteners of the present disclosure may possess any other geometry or configuration which permits insertion into tissue but resists removal therefrom. In embodiments the geometry or configuration permitting insertion may be the temporary shape, while the geometry or configuration resisting removal may be the permanent shape.

Suitable shape memory polymeric materials which may be utilized in a surgical fastener of the present disclosure include, for example, polyurethanes, poly(styrenebutadiene) block copolymers, polynorbornenes, caprolactones, dioxanones, diol esters including oligo (epsilon caprolactone) diol, lactic acid, lactide, glycolic acid, glycolide, ether-ester diols including oligo (p-dioxanone) diol, carbonates including trimethylene carbonate, combinations thereof, and the like. In embodiments, the shape memory polymer may be a copolymer of two components with different thermal characteristics, such as oligo (epsilon-caprolactone) dimethacrylates and butyl acrylates, including poly(epsilon-caprolactone) dimethacrylate-poly (n-butyl acrylate), or a diol ester and an ether-ester diol such as oligo (epsilon caprolactone) diol/oligo (p-dioxanone) diol copolymers. These multi-block oligo (epsilon-caprolactone) diol/oligo (p-dioxanone) diol copolymers possess two block segments: a "hard" segment and a "switching" segment linked together in linear chains. Such materials are disclosed, for example, in Lendlein, "Shape Memory Polymers-Biodegradable Sutures," Materials World, Vol. 10, no. 7, pp. 29-30 (July 2002).

In other embodiments, blends of bioabsorbable materials may be utilized including, but not limited to, urethanes blended with lactic acid and/or glycolic acid, homopolymers thereof or copolymers thereof, and acrylates blended with caprolactones such as polycaprolactone dimethacrylate poly(butyl acrylate) blends, and combinations thereof.

Other examples of suitable shape memory polymers and means for forming permanent and temporary shapes therewith are set forth in Lendlein et al., "Shape memory polymers as stimuli-sensitive implant materials," Clinical Hemorheology and Microcirculation, 32 (2005) 105-116, and Lendlein et al., "Biodegradable, Elastic Shape memory Polymers for Potential Biomedical Applications," Science, Vol. 269 (2002) 1673-1676.

In embodiments, a molding process may be utilized to produce the surgical fastener of the present disclosure. Plastic molding methods are within the purview of those skilled in the art and include, but are not limited to, melt molding, solution molding, and the like. Injection molding, extrusion molding, compression molding and other methods can also be used as the melt molding technique. Once placed in the mold with the proper dimensions and configuration, the polymeric material used to form the surgical fastener may be heated to a suitable temperature, referred to as the permanent temperature (Tₚₑᵣₘ) which may, in embodiments, be the melting temperature of the shape memory polymeric material utilized to form the surgical fastener. Heating of the surgical fastener may be at suitable temperatures including, for example, from about 40°C to about 180°C, in embodiments from about 80°C to about 150°C, for a period of time of from about 10 minutes to about 60 minutes, in embodiments from about 15 minutes to about 20 minutes, to obtain the permanent shape and dimensions, including its desired curvature.

In embodiments, a fastener of the present disclosure may have a helical configuration as its temporary shape, its permanent shape, or both. In other embodiments, each end of the fastener may adopt a helical or curved configuration as its permanent shape while the middle portion of the fastener may retain a straight configuration.

In other embodiments each end of a surgical fastener of the present disclosure may adopt an enlarged cross-sectional profile as its permanent shape while the middle portion of the fastener may retain a straight and, in embodiments, a narrow configuration.

After the surgical fastener with the desired curvature has been formed, the surgical fastener may be deformed at a deforming temperature to obtain, in embodiments, a straight temporary shape. Suitable temperatures for deformation will vary depending on the shape memory polymer utilized, but generally may be above the transition temperature of the polymer (Tₜᵣₐₙₛ), but below the Tₚₑᵣₘ. In embodiments, the shape memory polymer may be cooled from its Tₚₑᵣₘ to a lower temperature which remains above the Tₜᵣₐₙₛ and deformed, in embodiments by hand, to a straight temporary shape. In other embodiments, the surgical fastener may be straightened and cooled to room temperature (about 20° C to about 25° C) to obtain its straight temporary shape, although the temperature may differ depending upon the particular polymer employed. The surgical fastener may then be cooled to a temperature below the Tₜᵣₐₙₛ of the material utilized to form the fastener, at which time the surgical fastener of the present disclosure is ready for use. As the Tₜᵣₐₙₛ is usually greater than room temperature, in embodiments cooling to room temperature may be sufficient to form the temporary shape.

There are no particular limitations on the manner in which the deformation can be achieved. Deformation can be achieved either by hand or by means of a suitable device selected to provide the desired temporary straightness to the surgical fastener.

In order to keep the shape of the surgical fastener in its temporary shape, the shape memory surgical fastener of the present disclosure should be stored at a temperature which will not cause plastic deformation of the polymers. In embodiments, the shape memory surgical fastener may be stored in a refrigerator.

The surgical fasteners thus prepared recover their originally memorized curved or helical shape on heating, either by placement in a patient's body, or the addition of exogenous heat at a prescribed temperature, in embodiments above the Tₜᵣₐₙₛ of the shape memory polymer utilized. As the surgical fasteners of the present disclosure are utilized in a living body, heating with body heat (about 37° C) is possible. In such a case, the temperature for shape memorization should be as low as possible. In embodiments, recovery of the permanent shape may occur from about 1 second to about 5 seconds after insertion into tissue.

However, in some embodiments a higher shape memory temperature may be desirable in order to make the shape recover at a slightly higher temperature than body temperature. Thus, in some cases, releasing the surgical fastener from deformation to recover the originally memorized curved or helical shape can be achieved by heating. On heating at a temperature of from about 30° C to about 50° C, in embodiments from about 39° C to about 43° C, the temporary thin shape may be released and the memorized permanent curved or helical shape recovered. The higher the temperature for heating, the shorter the time for recovery of the originally memorized shape. The means for this heating is not limited. Heating can be accomplished by using a gas or liquid heating medium, heating devices, ultrasonic waves, electrical induction, and the like. Of course, in an application involving a living body, care must be taken to utilize a heating temperature which will not cause burns. Examples of liquid heating media include, physiological saline solution, alcohol, combinations thereof, and the like.

Similarly, in other embodiments, electrically active polymers, also known as electroactive polymers, which can alter their configuration upon application of electricity, may be utilized to fashion anchoring means to secure a cannula within the body. Suitable examples of electroactive polymers include poly(aniline), substituted poly(aniline)s, polycarbazoles, substituted polycarbazoles, polyindoles, poly(pyrrole)s, substituted poly(pyrrole)s, poly(thiophene)s, substituted poly(thiophene)s, poly(acetylene)s, poly(ethylene dioxythiophene)s, poly(ethylenedioxypyrrole)s, poly(p-phenylene vinylene)s, and the like, or combinations including at least one of the foregoing electroactive polymers. Blends or copolymers or composites of the foregoing electroactive polymers may also be used.

Similar to the change in shape which a shape memory material may undergo upon the application of energy, such as heat, in embodiments an electroactive polymer may undergo a change in shape upon the application of electricity from a low voltage electrical source (such as a battery). Suitable amounts of electricity which may be applied to effect such change will vary with the electroactive polymer utilized, but can be from about 5 volts to about 30 volts, in embodiments from about 10 volts to about 20 volts. The application of electricity will result in the fastener constructed of the electroactive polymer changing its shape into a fastening configuration.

While an electroactive polymer does not have the same permanent shape and temporary shape as those terms are described above with respect to shape memory polymers, as used herein the term "permanent shape" as applied to an electroactive polymer means, in embodiments, the shape the electroactive polymer adopts upon the application of electricity, and the term "temporary shape" as applied to an electroactive polymer means, in embodiments, the shape of the electroactive polymer adopts in the absence of electricity.

In embodiments, the surgical fastener of the present disclosure made of a shape memory polymeric material may be bioabsorbable. Times for absorption can vary from about 5 days to about 2 years after implantation, in embodiments from about 1 week to about 3 months after implantation, depending on the composition of the surgical fastener, its shape, and the like.

Mechanisms for introducing a fastener of the present disclosure into tissue during a surgical procedure are similar to known fastener introducers. The fastener of the present disclosure may be introduced into tissue and/or a medical device, including a mesh, by any means within the purview of those skilled in the art, including by hand or by conventional mechanical devices. In embodiments, an apparatus similar to the apparatus disclosed in U.S. Patent Application Publication No. 2003/0236534 , may be utilized to introduce a fastener of the present disclosure in vivo.

There is also disclosed a method of securing tissue, or a device such as a mesh to tissue, which includes providing the fastener of the present disclosure. The fastener may be inserted into tissue utilizing any method or means within the purview of those skilled in the art. In embodiments, a fastener may be inserted endoscopically or laparoscopically through a mesh into tissue and heated, in embodiments by body heat, in other embodiments by exogenous heat, whereupon the fastener reverts to its permanent helical shape and is able to adhere tissue together or a device, such as a mesh, to tissue. In other embodiments, where the fastener is made of an electroactive polymer, the fastening configuration of the fastener may be adopted upon the application of electricity. The number of surgical fasteners necessary to adhere tissue, or affix a device such as a mesh to tissue, will vary depending upon the configuration and length of the wound or defect, such as a hernia, to be repaired.

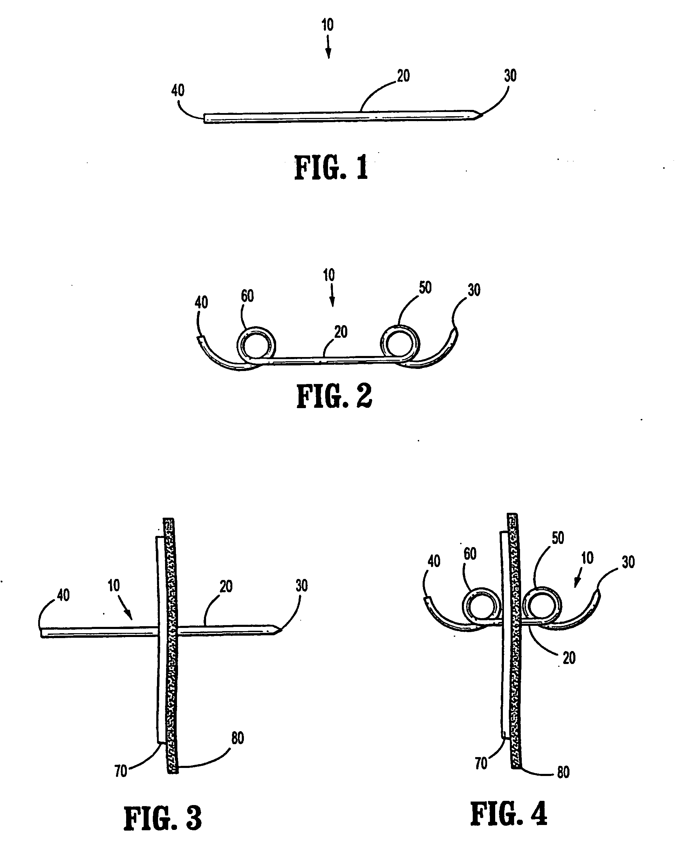

For example, as depicted in FIG. 1, surgical fastener 10 generally includes an elongated shaft 20. FIG. 1 is a depiction of a surgical fastener of the present disclosure in its straight temporary shape, prior to use. Surgical fastener 10 may be formed of shape memory polymeric materials as described above. Shaft 20 has a distal end 30 having a point and a proximal end 40 which may be a blunt end. In other embodiments, proximal end 40 may also be pointed (not shown).

As depicted in FIG. 2, once heated, either by body heat or the application of exogenous heat, surgical fastener 10 adopts its permanent shape, in embodiments a linear shaft 20 with distal end 30 curling to form a helical protrusion 50 and proximal end 40 similarly curling to form helical protrusion 60. The formation of helical protrusions 50 and 60 assist in anchoring surgical fastener 10 to tissue.

Use of a fastener of the present disclosure is now described with reference to FIGs. 3 and 4. Prior to use, surgical fastener 10 is of a temporary straight configuration as depicted in FIG. 1. As depicted in FIG. 3, pointed distal end 30 of surgical fastener 10 may be advanced through a pore or similar opening in a mesh 70, or in embodiments may puncture mesh 70, which may be utilized to repair a hernia. Distal end 30 of surgical fastener 10 may then be advanced through tissue 80, so that distal end 30 and proximal end 40 of surgical fastener 10 are on opposite sides of the combination of mesh 70 and tissue 80.

The heat of the patient, or the application of heat from an exogenous source, may then be used to heat surgical fastener 10 whereby it recovers its curved or helical, permanent shape. In embodiments, the heat of the body (about 37° C), may be sufficient for the surgical fastener to return to its curved or helical, permanent shape. In other embodiments, heat may be applied to the surgical fastener, in embodiments from about 39° C to about 43° C (just above human body temperature), to enhance the return of the shape memory polymer to its permanent shape. In other embodiments where the fastener is made of an electroactive polymer, the application of electricity may cause the fastener to recover its permanent fastening shape.

As depicted in FIG. 4, upon the application of heat, distal end 30 curls to form helical protrusion 50 and proximal end 40 similarly curls to form helical protrusion 60. Helical protrusion 50 remains on the tissue side of the mesh/tissue combination, while helical protrusion 60 remains on the mesh side of the mesh/tissue combination, so that helical protrusion 50 and helical protrusion 60 attach mesh 70 to tissue 80. As noted above, the number of surgical fasteners necessary to affix a mesh to tissue will vary depending upon the configuration and length of the wound or defect, such as a hernia, to be repaired.

While the figures depict surgical fasteners adopting helical configurations to attach meshes to tissue, it will be readily apparent to those skilled in the art that surgical fasteners may adopt alternate configurations to thereby secure a mesh or similar device to tissue, as well as to affix tissue to tissue. Such configurations include, for example, annular or circular configurations, spiral or corkscrew configurations, bent configurations, and the like. In embodiments, the configuration may include any permanent shape which secures tissue or a mesh or similar device to tissue. In some embodiments, a permanent shape having a larger cross-section at the ends may be sufficient.

In addition, while the figures depict surgical fasteners which pass through tissue and a device adjacent thereto a single time, in embodiments, a surgical fastener may pass through a mesh and tissue multiple times as it reverts from its temporary shape to its permanent shape. In this embodiment, it may be advantageous for proximal end 40 of surgical fastener 10 to have a point similar to the one depicted for distal end 30. In this embodiment, similar to the application described with FIGS. 3 and 4 above, the distal end of a fastener is passed through a mesh and tissue. As heat is applied to the surgical fastener in vivo, and as distal end 30 curls to adopt its helical shape, it may pass through the tissue and mesh a second time at a different location, and then pass again through the mesh and tissue a third time, in embodiments even more times, so that the resulting helix formed has portions on the mesh side of the mesh/tissue combination and portions on the tissue side of the mesh/tissue combination. Similarly, while not required, where proximal end 40 is similarly pointed, upon heating, as proximal end 40 curls to adopt its helical shape, it may pass through the mesh and tissue at a different location than the one where distal end 30 passed through mesh and tissue, and proximal end 40 may then pass again through the tissue and mesh a second time so that the helix formed has portions on the mesh side of the mesh/tissue combination and portions on the tissue side of the mesh/tissue combination.

In embodiments, it may be desirable to add optional ingredients, including medicinal agents, to the shape memory polymers utilized to form surgical fasteners of the present disclosure. The term "medicinal agent", as used herein, is used in its broadest sense and includes any substance or mixture of substances that have clinical use. Consequently, medicinal agents may or may not have pharmacological activity per se, e.g., a dye. Alternatively a medicinal agent could be any agent which provides a therapeutic or prophylactic effect, a compound that affects or participates in tissue growth, cell growth, cell differentiation, a compound that may be able to invoke a biological action such as an immune response, or could play any other role in one or more biological processes.

Examples of classes of medicinal agents which may be utilized in accordance with the present disclosure include antimicrobials; analgesics; antipyretics; anesthetics; antiepileptics; antihistamines; anti-inflammatory agents such as hormonal agents, hydrocortisone, prednisolone, prednisone, non-hormonal agents, allopurinol, indomethacin, phenylbutazone and the like; cardiovascular agents such as coronary vasodilators and nitroglycerin; diagnostic agents; sympathomimetics; cholinomimetics; antimuscarinics; antispasmodics; hemostats to halt or prevent bleeding; muscle relaxants; adrenergic neuron blockers; antineoplastics; immunogenic agents; immunosuppressants; gastrointestinal drugs; diuretics; steroids; lipids; lipopolysaccharides; polysaccharides; and enzymes. It is also intended that combinations of medicinal agents may be used.

Other medicinal agents which may be included with the shape memory polymers utilized to form the surgical fasteners of the present disclosure include local anesthetics; non-steroidal antifertility agents; parasympathomimetic agents; psychotherapeutic agents; tranquilizers; sedative hypnotics; sulfonamides; vaccines; vitamins; antimalarials; anti-migraine agents; anti-parkinson agents such as L-dopa; antispasmodics; anticholinergic agents (e.g. oxybutynin); bronchodilators; alkaloids; narcotics such as codeine, dihydrocodeinone, meperidine, morphine and the like; non-narcotics such as salicylates, aspirin, acetaminophen, d-propoxyphene and the like; opioid receptor antagonists, such as naltrexone and naloxone; anticoagulants; anticonvulsants; antidepressants; anti-emetics; prostaglandins and cytotoxic drugs; estrogens; antibiotics; anti-fungals; anti-virals; and immunological agents.

Suitable antimicrobial agents which may be included as a medicinal agent with the shape memory polymers utilized to form the surgical fasteners of the present disclosure include triclosan, also known as 2,4,4'-trichloro-2'-hydroxydiphenyl ether, chlorhexidine and its salts, including chlorhexidine acetate, chlorhexidine gluconate, chlorhexidine hydrochloride, and chlorhexidine sulfate, silver and its salts, including silver acetate, silver benzoate, silver carbonate, silver citrate, silver iodate, silver iodide, silver lactate, silver laurate, silver nitrate, silver oxide, silver palmitate, silver protein, and silver sulfadiazine, polymyxin, tetracycline, aminoglycosides, such as tobramycin and gentamicin, rifampicin, bacitracin, neomycin, chloramphenicol, miconazole, quinolones such as oxolinic acid, norfloxacin, nalidixic acid, pefloxacin, enoxacin and ciprofloxacin, penicillins such as oxacillin and pipracil, nonoxynol 9, fusidic acid, cephalosporins, and combinations thereof. In addition, antimicrobial proteins and peptides such as bovine lactoferrin and lactoferricin B may be included as a medicinal agent with the shape memory polymers utilized to form the surgical fasteners of the present disclosure.

Examples of hemostat materials which can be employed include fibrin-based, collagen-based oxidized regenerated cellulose-based, and gelatin-based topical hemostats. Examples of commercially available hemostat materials include fibrinogen-thrombin combination materials sold under the trade designations COSTASIS^{™} by Tyco Healthcare Group, LP, and TISSEEL^{™} sold by Baxter International, Inc. Hemostats herein also include astringents, for example, aluminum sulfate, and coagulants.

Other examples of suitable medicinal agents which may be included with the shape memory polymers utilized to form the surgical fasteners of the present disclosure include viruses and cells, peptides, polypeptides and proteins, analogs, muteins, and active fragments thereof, such as immunoglobulins, antibodies, cytokines (e.g. lymphokines, monokines, chemokines), blood clotting factors, hemopoietic factors, interleukins (IL-2, IL-3, IL-4, IL-6), interferons (β-IFN, (α-IFN and γ-IFN), erythropoietin, nucleases, tumor necrosis factor, colony stimulating factors (e.g., GCSF, GM-CSF, MCSF), insulin, anti-cancer and/or anti-tumor agents and tumor suppressors, blood proteins, gonadotropins (e.g., FSH, LH, CG, etc.), hormones and hormone analogs (e.g., growth hormone), vaccines (e.g., tumoral, bacterial and viral antigens); somatostatin; antigens; blood coagulation factors; growth factors (e.g., nerve growth factor, insulin-like growth factor); protein inhibitors, protein antagonists, and protein agonists; nucleic acids, such as antisense molecules, DNA and RNA; oligonucleotides; and ribozymes.

A single medicinal agent may be utilized with the shape memory polymers utilized to form the surgical fasteners of the present disclosure or, in alternate embodiments, any combination of medicinal agents may be utilized with the shape memory polymers utilized to form the surgical fasteners of the present disclosure.

The medicinal agent may be disposed on a surface of a surgical fastener of the present disclosure or impregnated in or combined with the shape memory polymers utilized to form the surgical fasteners of the present disclosure.

Additionally, where a shape memory polymer utilized to form a fastener of the present disclosure is bioabsorbable, an enzyme may be added to the shape memory polymers utilized to form the surgical fasteners of the present disclosure to increase their rate of degradation. Suitable enzymes include, for example, peptide hydrolases such as elastase, cathepsin G, cathepsin E, cathepsin B, cathepsin H, cathepsin L, trypsin, pepsin, chymotrypsin, γ-glutamyltransferase (γ-GTP) and the like; sugar chain hydrolases such as phosphorylase, neuraminidase, dextranase, amylase, lysozyme, oligosaccharase and the like; oligonucleotide hydrolases such as alkaline phosphatase, endoribonuclease, endodeoxyribonuclease and the like. In some embodiments, where an enzyme is added, the enzyme may be included in a liposome or microsphere to control the rate of its release, thereby controlling the rate of degradation of the surgical fastener of the present disclosure. Methods for incorporating enzymes into liposomes and/or microspheres are within the purview of those skilled in the art.

The present disclosure provides a surgical fastener for use in surgery. The surgical fastener is made of a shape memory polymer which alters its shape in vivo to assist in securing tissue or devices, such as meshes used in hernia repair, to tissue.

## Claims

1. A surgical fastener (10) comprising:
an elongated shaft (20) formed of a shape memory polymeric material having a pointed distal end (30), and a proximal end (40),
wherein:
the surgical fastener (10) possesses a temporary shape permitting insertion of the surgical fastener (10) into tissue and a permanent shape capable of affixing the surgical fastener (10) to tissue and an optional medical device,
**characterized in that**
in the temporary shape, the surgical fastener (10) is straight, and
in the permanent shape, the surgical fastener (10) has two annular or circular shapes (50, 60) spaced by a straight shaft.

2. The surgical fastener (10) of claim 1, wherein the shape memory polymer is selected from the group consisting of polyurethanes, poly(styrene-butadiene) block copolymers, polynorbornenes, caprolactones, dioxanones, diol esters, ether-ester diols, carbonates, and combinations thereof.

3. The surgical fastener (10) of claim 1, wherein the shape memory polymer is selected from the group consisting of oligo (epsilon caprolactone) diol, lactic acid, lactide, glycolic acid, glycolide, oligo (p-dioxanone) diol, trimethylene carbonate, and combinations thereof.

4. The surgical fastener (10) of claim 1, wherein the shape memory polymer is selected from the group consisting of poly(styrene-butadiene) copolymers, oligo (epsilon caprolactone) diol/oligo (p-diozanone) diol copolymers, and poly(epsilon- caprolactone) dimethacrylate-poly(n-butyl acrylate) copolymers.

5. The surgical fastener (10) of claim 1, wherein the shape memory polymer comprises a blend of materials selected from the group consisting of urethanes, lactic acid, glycolic acid, acrylates, caprolactones, homopolymers thereof, copolymers thereof, and combinations thereof.

6. The surgical fastener (10) of claim 1, wherein the shape memory polymer undergoes a change in shape at a temperature from 30°C to 50°C.

7. The surgical fastener (10) of claim 1, wherein the shape memory polymer undergoes a change in shape upon the application of electricity.

8. The surgical fastener (10) of claim 7, wherein the shape memory polymer is selected from the group consisting of poly(aniline), substituted poly(aniline)s, polycarbazoles, substituted polycarbazoles, polyindoles, poly(pyrrole)s, substituted poly(pyrrole)s, poly(thiophene)s, substituted poly(thiophene)s, poly(acetylene)s, poly(ethylene dioxythiophene)s, poly(ethylenedioxypyrrole)s, poly(p-phenylene vinylene)s, and combinations thereof.

9. The surgical fastener (10) of claim 1, wherein the shape memory polymer is bioabsorbable.

10. The surgical fastener (10) of claim 9, wherein the shape memory polymer is absorbed over a period of from 5 days to 2 years after implantation.

11. The surgical fastener (10) of claim 9, wherein the shape memory polymer is absorbed over a period of from 1 week to 3 months after implantation.

12. The surgical fastener (10) of claim 1, wherein the optional medical device comprises a surgical mesh (70).

13. The surgical fastener (10) of claim 1, further comprising a medicinal agent.

14. The surgical fastener (10) of claim 1, wherein the temporary shape is released and the permanent shape is recovered at a temperature of from 30°C to 50°C.

15. The surgical fastener (10) of claim 14, wherein the temporary shape is released and the permanent shape is recovered at a temperature of from 39°C to 43°C.

## Patentansprüche

1. Chirurgisches Befestigungselement (10), das Folgendes aufweist:
einen langgestreckten Schaft (20), der aus einem Formgedächtnis-Polymermaterial gebildet ist, welcher ein spitzes distales Ende (30) und ein proximales Ende (40) aufweist,
wobei:
das chirurgische Befestigungselement (10) eine vorübergehende Form, welche die Einführung von dem chirurgischen Befestigungselement (10) in das Gewebe ermöglicht, und eine dauerhafte Form besitzt, die es ermöglicht, das chirurgische Befestigungselement (10) an Gewebe und einer optionalen medizinischen Vorrichtung zu befestigen,
**dadurch gekennzeichnet, dass**
in der vorübergehenden Form das chirurgische Befestigungselement (10) gerade ist, und
in der dauerhaften Form das chirurgische Befestigungselement (10) zwei ringförmige oder kreisförmige Formen (50, 60) aufweist, die durch einen geraden Schaft beabstandet sind.

2. Chirurgisches Befestigungselement (10) nach Anspruch 1, wobei das Formgedächtnispolymer ausgewählt ist aus der Gruppe, die aus Polyurethanen, Poly(StyrolButadien)-Blockcopolymeren, Polynorbornenen, Caprolactonen, Dioxanonen, Diolestern, Ether-Ester-Diolen, Carbonaten und Kombinationen davon besteht.

3. Chirurgisches Befestigungselement (10) nach Anspruch 1, wobei das Formgedächtnispolymer ausgewählt ist aus der Gruppe, die aus Oligo-(epsilon-caprolacton)-diol, Milchsäure, Lactid, Glykolsäure, Glycolid, Oligo-(p-dioxanon)-diol, Trimethylencarbonat und Kombinationen davon besteht.

4. Chirurgisches Befestigungselement (10) nach Anspruch 1, wobei das Formgedächtnispolymer ausgewählt ist aus der Gruppe, die aus Poly(Styrol-Butadien)-Copolymeren, Oligo-(epsilon-caprolacton)-diol/Oligo-(p-dioxanon)-diol-Copolymere und Poly-(epsilon- caprolacton)-dimethacrylat-poly(n-butylacrylat)-Copolymeren besteht.

5. Chirurgisches Befestigungselement (10) nach Anspruch 1, wobei das Formgedächtnispolymer eine Mischung von Materialien umfasst, die ausgewählt sind aus der Gruppe, die aus Urethanen, Milchsäure, Glykolsäure, Acrylaten, Caprolactonen, Homopolymeren davon, Copolymeren davon und Kombinationen davon besteht.

6. Chirurgisches Befestigungselement (10) nach Anspruch 1, wobei das Formgedächtnispolymer eine Änderung der Form bei einer Temperatur von 30 °C bis 50 °C erfährt.

7. Chirurgisches Befestigungselement (10) nach Anspruch 1, wobei das Formgedächtnispolymer eine Änderung der Form durch die Anwendung von elektrischem Strom erfährt.

8. Chirurgisches Befestigungselement (10) nach Anspruch 7, wobei das Formgedächtnispolymer ausgewählt ist aus der Gruppe, die aus Poly(anilin), substituierten Poly(anilinen), Polycarbazolen, substituierten Polycarbazolen, Polyindolen, Poly(pyrrolen), substituierten Poly(pyrrolen), Poly(thiophenen), substituierten Poly(thiophenen), Poly(acetylenen), Poly(ethylen-dioxythiophenen), Poly(ethylen-dioxypyrrolen), Poly(p-phenylen-vinylenen) und Kombinationen davon besteht.

9. Chirurgisches Befestigungselement (10) nach Anspruch 1, wobei das Formgedächtnispolymer biologisch resorbierbar ist.

10. Chirurgisches Befestigungselement (10) nach Anspruch 9, wobei das Formgedächtnispolymer über einen Zeitraum von 5 Tagen bis 2 Jahre nach der Implantation resorbiert wird.

11. Chirurgisches Befestigungselement (10) nach Anspruch 9, wobei das Formgedächtnispolymer über einen Zeitraum von 1 Woche bis 3 Monate nach der Implantation resorbiert wird.

12. Chirurgisches Befestigungselement (10) nach Anspruch 1, wobei die optionale medizinische Vorrichtung ein chirurgisches Netz (70) ist.

13. Chirurgisches Befestigungselement (10) nach Anspruch 1, das ferner ein medizinisches Mittel umfasst.

14. Chirurgisches Befestigungselement (10) nach Anspruch 1, wobei bei einer Temperatur von 30 °C bis 50 °C die vorübergehende Form freigegeben und die dauerhafte Form wiederhergestellt wird.

15. Chirurgisches Befestigungselement (10) nach Anspruch 14, wobei bei einer Temperatur von 39 °C bis 43 °C die vorübergehende Form freigegeben und die dauerhafte Form wiederhergestellt wird.

## Revendications

1. Attache chirurgicale (10) comprenant :
une tige allongée (20) formée d'un matériau polymère à mémoire de forme ayant une extrémité distale pointue (30) et une extrémité proximale (40),
dans laquelle :
l'attache chirurgicale (10) possède une forme temporaire permettant l'insertion de l'attache chirurgicale (10) dans un tissu et une forme permanente capable de fixer l'attache chirurgicale (10) au tissu et à un dispositif médical facultatif,
**caractérisée en ce que** :
dans la forme temporaire, l'attache chirurgicale (10) est droite, et
dans la forme permanente, l'attache chirurgicale (10) présente deux formes annulaires ou circulaires (50, 60) espacées par une tige droite.

2. Attache chirurgicale (10) selon la revendication 1, dans laquelle le polymère à mémoire de forme est choisi dans le groupe constitué des polyuréthanes, des copolymères séquencés de poly(styrène-butadiène), des polynorbornènes, des caprolactones, des dioxanones, des esters de diols, des diols d'éther-ester, des carbonates et de leurs combinaisons.

3. Attache chirurgicale (10) selon la revendication 1, dans laquelle le polymère à mémoire de forme est choisi dans le groupe constitué d'un oligo(epsilon caprolactone)diol, de l'acide lactique, d'un lactide, de l'acide glycolique, d'un glycolide, d'un oligo(p-dioxanone)diol, du carbonate de triméthylène et de leurs combinaisons.

4. Attache chirurgicale (10) selon la revendication 1, dans laquelle le polymère à mémoire de forme est choisi dans le groupe constitué des copolymères de poly(styrène-butadiène), des copolymères d'oligo(epsilon caprolactone)diol/oligo(p-diozanone)diol et des copolymères de diméthacrylate de poly(epsilon-caprolactone) et de poly(n-butyl acrylate).

5. Attache chirurgicale (10) selon la revendication 1, dans laquelle le polymère à mémoire de forme comprend un mélange de matériaux choisis dans le groupe constitué des uréthanes, de l'acide lactique, de l'acide glycolique, des acrylates, des caprolactones, de leurs homopolymères, de leurs copolymères et de leurs combinaisons.

6. Attache chirurgicale (10) selon la revendication 1, dans laquelle le polymère à mémoire de forme subit un changement de forme à une température de 30 °C à 50 °C.

7. Attache chirurgicale (10) selon la revendication 1, dans laquelle le polymère à mémoire de forme subit un changement de forme lors de l'application d'électricité.

8. Attache chirurgicale (10) selon la revendication 7, dans laquelle le polymère à mémoire de forme est choisi dans le groupe constitué de la poly(aniline), des poly(aniline)s substituées, des poly(carbazole)s, des poly(carbazole)s substitués, des poly(indoles), des poly(pyrrole)s, des poly(pyrrole)s substitués, des poly(thiophène)s, des poly(thiophène)s substitués, des poly(acétylène)s, des poly(éthylènedioxythiophène)s, des poly(éthylène-dioxypyrrole)s, des poly(p-phénylène vinylène)s et de leurs combinaisons.

9. Attache chirurgicale (10) selon la revendication 1, dans laquelle le polymère à mémoire de forme est bioabsorbable.

10. Attache chirurgicale (10) selon la revendication 9, dans laquelle le polymère à mémoire de forme est absorbé sur une période de 5 jours à 2 ans après implantation.

11. Attache chirurgicale (10) selon la revendication 9, dans laquelle le polymère à mémoire de forme est absorbé sur une période de 1 semaine à 3 mois après implantation.

12. Attache chirurgicale (10) selon la revendication 1, dans laquelle le dispositif médical facultatif comprend un treillis chirurgical (70).

13. Attache chirurgicale (10) selon la revendication 1, comprenant en outre un agent médical.

14. Attache chirurgicale (10) selon la revendication 1, dans laquelle la forme temporaire est libérée et la forme permanente est recouvrée à une température de 30 °C à 50°C.

15. Attache chirurgicale (10) selon la revendication 14, dans laquelle la forme temporaire est libérée et la forme permanente est recouvrée à une température de 39 °C à 43°C.
